# EUROPEAN PATENT SPECIFICATION

(11) **EP 1 051 361 B1**
(45) Date of publication and mention of the grant of the patent: **17.03.2004**
(21) Application number: 99903817.7
(22) Date of filing: 02.02.1999
(51) Int. Cl.: C02F 3/10, C12N 11/00

(54) **WASTE WATER TREATMENT, MEDIA THEREFOR AND ITS MANUFACTURE**
ABWASSERBEHANDLUNG, TRÄGERMATERIAL DAFÜR UND SEINE HESTELLUNG
TRAITEMENT DES EAUX USEES, MILIEU DESTINE AUDIT TRAITEMENT ET SON PROCEDE DE FABRICATION

(30) Priority: 04.02.1998 GB 9802388
(43) Date of publication of application: 15.11.2000
(73) Proprietor: Todd, John James, Alton, Hampshire GU34 1DT (GB)
(72) Inventor: TODD, John, James, Alton Hampshire GU34 1DT (GB); HOPWOOD, Adrian, Childrey, Wantage Oxfordshire OX12 9UP (GB)
(74) Representative: Rees, Alexander Ellison
(86) International application number: PCT/GB1999/000346
(87) International publication number: WO 1999/040035

(56) References cited:
- WO-A-86/05811
- WO-A-95/17357
- WO-A-96/25367
- US-A- 4 576 718
- US-A- 4 915 884

## Description

This invention relates to loose particulate material for use in waste water treatment plant and methods, to the manufacture of such material, and to methods and apparatus for the treatment of waste water using such material.

Waste water may be treated by gasification, for example by the aeration or oxygenation of sewage or other waste water containing organic matter degradable by the action of oxygen thereon. A wide range of treatment methods and apparatus has been used and proposed. Oxygen does not dissolve easily or quickly in water and it is therefore in principle desirable to utilize fine bubble aerators wherein the bubbles are less than 2 mm and desirably less than 1 mm in diameter. Smaller bubbles have a larger specific surface area for oxygen transfer into the liquid, and also rise more slowly through the liquid to give a longer time for the oxygen to transfer before the bubble reaches the liquids surface. Treatment plants are known comprising a treatment vessel with aerator devices submerged in the waste water to produce the bubbles.

It has also been proposed to provide a treatment plant where the treatment vessel contains a bed of loose material. Aeration then causes a degree of fluidization of the bed and sustains the growth of a population of microorganisms on the material of the bed. In the presence of dissolved oxygen the microorganisms convert the organic matter in the waste water to carbon dioxide, water and to more bulky cellular materials and sludge thus alleviating the biological oxygen demand (BOD). Under appropriate operating conditions they will also convert ammonia to nitrate compounds. The surplus sludge thus formed can pass out with the effluent for eventual separation and recycling if desired.

Problems of fouling and clogging of the aerator devices and any pipe work can be acute where they are buried or caged beneath a bed of loose material. Regular closing and draining of the plant for cleaning and unblocking or replacement of the aerators is inefficient and expensive due to the need also to move aside or remove the filter bed material to gain access to the buried aerators.

Our publication no. WO/95/17351 describes a method and apparatus for treatment of waste water wherein such problems are mitigated, and in particular discloses loose particulate material for use as a fluidizable bed in the waste water treatment, said material being characterized by particles of a substantially inert mineral adhered to, coated on or coated by plastics material to provide a habitat for microorganisms effective in waste water treatment.

That Publication discloses such loose particulate material having a density in the range of from substantially 1.0 to substantially 1.3 g/cc, having a specific surface area in excess of approximately 600 m² per cubic metre of the loose material, and having a particle size range of substantially 3 mm to substantially 10 mm in diameter. An example of the material is disclosed as particles of sand or other inert mineral particles at least partially adhered to, coated on or coated by plastics material, preferably a thermoplastics material such as polyethylene. It was disclosed that the material could be produced to a desired density for a particular application by changing the initial proportions of mineral and plastics.

Our Publication No. WO/96/25367 describes improved loose particulate material for use in waste water treatment. It discloses granules of plastics material having grains of an inert mineral such as sand coated thereon, the granules having a predetermined particle size range, and the grains having a predetermined particle size range and being disposed at a predetermined packing density range on the granules. The particles have an average density of approximately 1.0 g/cc such that a proportion tends to float and a proportion tends to sink in the waste water. The particles are manufactured by contacting the granules of plastics material with a mixture of grains of the inert mineral and grains of a soluble substance such as salt, at an elevated temperature, to coat the granules with the mixture, and subsequently dissolving the soluble substance grains from the coating to provide the granules coated with the grains of inert mineral in the predetermined packing density range.

Such loose particulate material has been found in practice to be effective for use in waste water treatment. Such materials have been found to provide a particularly suitable habitat for a high population density of microorganisms of the type effective in waste water treatment.

The inventors have also found that, by using significantly embedded grains, the subsequent step of dissolving away the soluble substance grains leaves concavities in the surface of the granules thereby aiding achievement of the desired high specific surface area.

An object of the present invention is to provide further improved material for waste water treatment and methods for its manufacture.

According to the present invention in one aspect there is provided loose particulate material for use in waste water treatment, said material characterised by granules of plastics material carrying weighting material so that the particles have an average density of approximately 1.0 g/cc, the weighting material being grains of sodium chloride and said weighting material being incorporated substantially wholly within the granules such that the weighting material is substantially not exposed at the surfaces of the granules, and the surfaces of the granules being provided with concavities to provide a habitat for microorganisms effective in waste water treatment.

An advantage of incorporating the grains of weighting material within the granules is that the grains can achieve the required weighting function to bring the average particle density to approximately 1.0 g/cc and reducing their likelihood of becoming gradually abraded away over an extended period of use. It is desirable for waste water treatment plants to function for periods of many years between major refits.

If sand grains are largely exposed on the surface of the granules, as with the particles described in our above-mentioned WO/96/25367, they may become abraded away over a period of use. The proportion of granules of lower density than aerated waste water would then be undesirably increased.

The present invention also provides a method of manufacture of loose particulate material for use in waste water treatment, said method comprising incorporating a weighting material within granules of plastics material so that the weighting material is carried within the granules and the particles have an average density of approximately 1.0 g/cc, and contacting the granules with grains of a soluble substance, at an elevated temperature, to coat the granules with the soluble substance grains, and subsequently dissolving the soluble substance grains from the coating to provide the surface of the granules with concavities to serve as a habitat for microorganisms effective in waste water treatment, the weighting material and the soluble substance grains both being grains of sodium chloride.

The granules are preferably contacted with the soluble substance grains at an elevated temperature such that the grains coat the granules by burying themselves significantly into a partially melted outer surface of the granules, whereby the step of dissolving the soluble substance grains leaves the desired concavities in the granules surface.

An embodiment of the invention will now be described. Granules of plastics material are provided. These are suitably granules of polyethylene and preferably have substantially uniform particle size. The particle size is suitably in the range 3 to 10 mm considered as a sieve size range. The preferred size range is 4 to 8 mm, preferably about 6 mm. The granules may be of approximately spherical, ellipsoidal or cylindrical shape, or may be of irregular shape such as commercially available recycled chips, for example granulated from moulded rejects. Flattened shapes have a greater specific surface area.

It is desired to achieve a high specific surface area for the loose particulate material, for example in excess of about 600 m² per cubic metre or even 2000 m² per cubic metre. The greater the specific surface area, the correspondingly larger is the habitat area for the microorganisms effective in the treatment.

The specific gravity of the plastics material is fixed by the choice of plastics material, suitably polyethylene, and is less than 1.0 g/cc. The granules carry weighting materials so that the particles have an average density of approximately 1.0 g/cc. Thus in use a proportion of particles tend to float and a proportion tend to sink in the waste water, but on average have neutral buoyancy so as to circulate easily as the waste water is aerated and circulated. The density is preferably a little less than 1.0 g/cc because aerated waste water itself has a density a little less than 1.0 g/cc.

The surface of the weighted granules is provided with concavities to serve as habitats for microorganisms effective in waste water treatment. The concavities preferably cover substantially the whole surface of the granules, thereby providing the greatest possible increase in effective surface area.

The concavities may be about 0.1 mm to 1 mm in effective width and in effective depth, preferably about 0.3 mm in width and a little less in depth. It will be appreciated that if such concavities cover substantially the whole surface of the granules then the specific surface area is increased by a factor of about 3 or more, also depending upon the general shape and regularity of the concavities themselves.

The concavities not only increase the specific surface area, and thus the available habitat area for the microorganisms, but they also provide a sheltered environment which particularly favours the growth of a desirably dense and stable population of microorganisms. In prior art arrangements with microorganisms exposed on the external surfaces of media or structures, they are liable to become detached by abrasion or tumbling during circulation of waste water.

A particular advantage of the particles of the present invention is that they are loose and of neutral buoyancy, and are thus continually mobile within and throughout the body of waste water within the treatment vessel. Thus, during circulation and aeration this ensures high efficiency of contact between the microorganisms and the waste water being treated.

However, an advantage of the invention can also be achieved in relation to other waste water treatment media, for example structures adapted to be fixed in position within a waste water treatment vessel. Such medium is also preferably a plastics material and has a surface similarly provided with said concavities to provide a habitat for microorganisms effective in waste water treatment.

The loose particulate material is suitably manufactured by incorporating the weighting material within the plastics material granules and then creating the surface concavities. The weighted granules are contacted with grains of a soluble substance, at an elevated temperature, to coat the weighted granules with the soluble substance grains, and subsequently dissolving the soluble substance grains from the coating to provide the surface of the granules with the concavities.

The soluble substance is common salt, i.e. sodium chloride. The salt grain size range is of course governed by the desired size of the eventual concavities and is suitably about 0.3 mm. Salt grains tend to be cubic crystals and thus the eventual concavities also tend to be generally cubic. This is advantageous in increasing the specific surface area.

The weighting material is identical to the soluble substance. Accordingly, both are common salt grains. The salt grains incorporated within the granules as weighting material would thereby tend to be protected from being dissolved away by the waste water.

It has been found possible to provide loose particulate material having a specific surface area considerably in excess of 600 m² per cubic metre, i.e. up to 3000 m² per cubic metre or 4000 m² per cubic metre. Moreover, the concavities provide a sheltered environment which particularly favours the growth of a desirably dense and stable population of microorganisms. The material finds use in waste water treatment.plants and methods for example as described in our above-mentioned WO/95/17351 or WO/96/25367.

## Claims

1. Loose particulate material for use in waste water treatment, said material **characterised by** granules of plastics material carrying weighting material so that the particles have an average density of approximately 1.0g/cc, the weighting material being grains of sodium chloride and said weighting material being incorporated substantially wholly within the granules such that the weighting material is substantially not exposed at the surfaces of the granules, and the surfaces of the granules being provided with concavities to provide a habitat for microorganisms effective in waste water treatment.

2. A method of manufacture of loose particulate material for use in waste water treatment, said method comprising incorporating a weighting material within granules of plastics material so that the weighting material is carried within the granules and the particles have an average density of approximately 1.0g/cc, and contacting the granules with grains of a soluble substance, at an elevated temperature, to coat the granules with the soluble substance grains, and subsequently dissolving the soluble substance grains from the coating to provide the surfaces of the granules with concavities to serve as a habitat for microorganisms effective in waste water treatment, the weighting material and the soluble substance grains both being grains of sodium chloride.

3. A waste water treatment method **characterised by** charging a treatment vessel with waste water and loose particulate material according to claim 1, and aerating the waste water by means of aerators.

## Patentansprüche

1. Loses teilchenförmiges Material für die Verwendung bei der Abwasserbehandlung, wobei das Material durch Granulat aus Kunststoffmaterial gekennzeichnet ist, das Beschwerungsmaterial derart trägt, dass die Teilchen eine durchschnittliche Dichte von ca. 1,0 g/cc aufweisen, wobei das Beschwerungsmaterial aus Körnern von Natriumchlorid besteht und das Beschwerungsmaterial im Wesentlichen vollständig in das Granulat derart eingearbeitet ist, dass das Beschwerungsmaterial im Wesentlichen an den Oberflächen des Granulats nicht freigelegt ist und die Oberflächen des Granulats mit Aushöhlungen ausgestattet sind für die Bereitstellung eines Lebensraums für bei der Abwasserbehandlung wirksame Mikroorganismen.

2. Methode für die Herstellung von losem teilchenförmigem Material für die Verwendung bei der Abwasserbehandlung, wobei die Methode Folgendes umfasst: Einarbeiten eines Beschwerungsmaterials innerhalb eines Granulats aus Kunststoffmaterial derart, dass das Beschwerungsmaterial innerhalb des Granulats getragen wird und die Teilchen eine durchschnittliche Dichte von ca. 1,0 g/cc aufweisen, und Kontaktieren des Granulats mit Körnern einer löslichen Substanz bei erhöhter Temperatur, um das Granulat mit den Körnern der löslichen Substanz zu beschichten, und darauf folgendes Herauslösen der Körner der löslichen Substanz aus der Beschichtung, um die Oberflächen des Granulats mit Aushöhlungen auszustatten, die als Lebensraum für Mikroorganismen dienen, die bei der Abwasserbehandlung wirksam sind, wobei sowohl das Beschwerungsmaterial als auch die Körner der löslichen Substanz aus Körnern aus Natriumchlorid bestehen.

3. Abwasserbehandlungsmethode, **gekennzeichnet durch** Füllen eines Behandlungsgefäßes mit Abwasser und losem teilchenförmigem Material nach Anspruch 1 und Durchlüften des Abwassers **durch** Belüfter.

## Revendications

1. Matériau particulaire en vrac pour utilisation dans le traitement des eaux résiduaires, ledit matériau étant **caractérisé par** des granulés de matériau plastique portant un matériau alourdissant, de telle sorte que les particules aient une densité moyenne d'approximativement 1,0 g/cc, le matériau alourdissant étant des grains de chlorure de sodium et ledit matériau alourdissant étant incorporé pour une part substantielle entièrement au sein des granulés de telle sorte que le matériau alourdissant ne soit pas substantiellement exposé aux surfaces des granulés, et les surfaces des granulés étant pourvues de cavités destinées à fournir un habitat pour des micro-organismes efficaces dans le traitement des eaux résiduaires.

2. Procédé de fabrication de matériau particulaire en vrac pour utilisation dans le traitement des eaux résiduaires, ledit procédé comprenant l'incorporation d'un matériau alourdissant au sein des granulés de matériau plastique, de telle sorte que le matériau alourdissant soit porté au sein des granulés et que les particules aient une densité moyenne d'approximativement 1,0 g/cc, et la mise en contact des granulés avec des grains d'une substance soluble, à une température élevée, pour revêtir les granulés à l'aide des grains de substance soluble et la dissolution subséquente des grains de substance soluble provenant du revêtement pour pourvoir les surfaces des granulés de cavités destinées à servir d'habitat à des micro-organismes efficaces dans le traitement des eaux résiduaires, le matériau alourdissant et les grains de substance soluble étant tous les deux des grains de chlorure de sodium.

3. Procédé de traitement des eaux résiduaires **caractérisé par** le chargement d'un récipient de traitement à l'aide d'eaux résiduaires et de matériau particulaire en vrac selon la revendication 1, et l'aération des eaux résiduaires au moyen de dispositifs d'aération.
